## Europäisches Patentamt

## European Patent Office

## Office européen des brevets

(11) Publication number: **0 127 153**

**A2**

(12)

# EUROPEAN PATENT APPLICATION

(21) Application number: **84105980.1**

(22) Date of filing: **25.05.84**

(51) Int. Cl.³: **A 61 K 39/112**
**A 61 K 39/108**
**//C12N15/00**

(30) Priority: **26.05.83 GB 8314645**

(43) Date of publication of application:
**05.12.84 Bulletin 84/49**

(84) Designated Contracting States:
**AT BE CH DE FR GB IT LI LU NL SE**

(71) Applicant: **THE WELLCOME FOUNDATION LIMITED**
**183-193 Euston Road**
**London NW1 2BP(GB)**

(71) Applicant: **SCHWEIZ. SERUM UND IMPFINSTITUT UND**
**INSTITUT ZUR ERFORSCHUNG VON**
**INFEKTIONSKRANKHEITEN**
**Rehhagstrasse 79**
**Bern(CH)**

(72) Inventor: **Dallas, Walter Southwick**
**205 East Green Drive**
**Raleigh North Carolina(US)**

(72) Inventor: **Germanier, Rene**
**Mettlenhoelzliweg 8**
**CH-3074 Muri B Bern(CH)**

(74) Representative: **Sandmair, Kurt, Dr. Dr. et al,**
**Patentanwälte Dr. Berg Dipl.-Ing. Stapf Dipl.-Ing.**
**Schwabe Dr. Dr. Sandmair Postfach 86 02 45**
**Stuntzstrasse 16**
**D-8000 München 86(DE)**

(54) Bivalent vaccines.

(57) The present invention relates to immunogenic preparations of attenuated strains of *S.typhi* which have been transformed by a plasmid containing the cistron coding for the LT toxin B sub-unit of *E.coli*, and which express said toxin sub-unit. These immunogenic preparation are useful for providing protection against typhoid and cholera or *E.coli* infections caused by the LT-toxin.

FIG.1

Bivalent Vaccines

The present invention relates to bivalent immunogenic preparations for use in vaccine formulations to provide protection against Salmonella typhi and Vibrio cholerae infections.

Vaccines which provide protection against typhoid fever and cholera infections respectively have been used extensively in the past for the prophylactic protection of people resident in, or travelling to, areas in which one or both of these diseases are endemic. However, such vaccines have been found to suffer from serious disadvantages, for example, with regard to the protection that they afford or the problems arising from administration including adverse reactions.

Typhoid vaccines which have been previously employed on a large scale have generally been based on killed whole cell S.typhi preparations which are administered by parenteral injection. However, such vaccines, while providing reasonably good protection, e.g. 75-80% over three years, have a number of side effects, e.g. pyrogenicity which may arise from the considerable proportion of endotoxin present in the preparations. It has also been proposed to use an oral killed typhoid vaccine but doubts have been expressed as to the effectiveness of such a vaccine. A different approach is the use of a live attenuated oral vaccine based on an S.typhi strain which, as a result of genetic modification, is not virulent but is effective in stimulating gastro-intestinal immunity. An example of such an approach is the S.typhi mutant strain Ty21a which has been used with good success in a field trial as an oral vaccine to prevent typhoid fever.

With regard to cholera vaccines, these have in the past been generally based on preparations of killed organisms for parenteral injection. Such vaccines have only provided limited protection, e.g. 50% over a period of 3-6 months. This limited protection is believed to be due to the fact that cholera arises from the local colonisation of the small intestine with the Vibrio cholera organism, i.e. it is not a systemic infection. Thus, the systemic administration of a vaccine formulation is not attacking the organism at its main site of action. A further problem with such vaccines is that they are similar to the typhoid vaccines discussed above in the occurrence of side-effects after injection.

In order to provide effective protection against cholera, it would be desirable to have available an orally administrable vaccine which would enable one to provide local protection in the small intestine. However, a problem with the development of such a vaccine is to find a vaccine which, while providing satisfactory MG/TJM/8th May, 1984

X234
0127153

protection, is safe to use. Various proposals have been made for oral cholera vaccines, for example, based on killed whole cell preparations, live attenuated strains and cholera toxin B-subunit formulations. However, of these proposals, only Texas Star, which is based on a live avirulent strain, has been subjected to clinical evaluation and this has been found to suffer from the disadvantage that it causes diarrhoea in 10-20% of the people to whom the vaccine is administered.

Reference has been made above to the possibility of using vaccines based on the cholera toxin B-subunit. Cholera toxin (CT) is a protein with two types of subunit, an A-subunit which is responsible for the toxic effect and the B- subunit which is responsible for cell binding. The latter subunit has been proposed as an immunogen for protection against cholera since it has been found that isolated antibodies to the B-subunit have cholera toxin-neutralising activity. Unfortunately, such vaccines do not have the colonization ability that is possessed by live cell vaccines.

The cholera toxin (CT) has been found to be similar in certain respects to the heat-labile toxin (LT) of the organism E.coli, the B-subunits of CT and LT being about 80% homologous in their amino acid sequence. These subunits are also the immunodominant moieties of their respective toxins and antibodies to these subunits will neutralise both the homologous and heterologous toxin.

We have now discovered that immunogenic preparations providing protection against both typhoid and cholera can be obtained by transforming an S.typhi strain by a plasmid containing a gene coding for the LT-B toxin subunit of E.coli. The resulting immunogenic preparation comprises an S.typhi organism providing protection against typhoid together with a plasmid containing a gene coding for the LT-B toxin subunit of E.coli, the said plasmid being capable of transforming the said S.typhi strain. In view of the successful use of oral vaccines based on attenuated strains of S.typhi for the prevention of typhoid, it is advantageous to use such strains for transformation by the above-mentioned plasmids. The present invention thus enables one to provide a bivalent vaccine from a single preparation which contains the antigenic determinants for protection against both typhoid and cholera.

MG/TJM/8th May, 1984

X234
0127153

The present invention thus avoids the need to prepare immunogenic preparations from two separate bacterial sources with consequent considerable savings in terms of fermentation, separation and purification resources. The use of a bivalent vaccine in accordance with the invention also enables one to prepare a unitary preparation in contrast to the practice previously necessary of administering the typhoid and cholera vaccines separately. The separate administration of such vaccines, while necessary to provide protection against both typhoid and cholera, could give rise to problems in regard to the side-effect arising from such vaccines, particularly those for parenteral administration discussed above.

According to a preferred feature of the present invention, we provide an immunogenic preparation suitable for protection against typhoid and cholera infections which comprises a live non-virulent S.typhi strain capable of providing protection against typhoid upon oral administration, together with a DNA vector which is capable of transforming the said strain and which contains a sequence of bases which substantially encode the LT-B toxin subunit of E.coli.

'DNA vector' as used herein means a vehicle composed of DNA which is capable of replication and expression in the desired strain of S.typhi especially when containing a sequence of foreign DNA. Such DNA vectors include for example plasmids, cosmids and bacteriophages, preferably plasmids and particularly pWD615.

The invention further provides a method of preparing an immunogenic preparation as described above which comprises transforming the said S.typhi strain by the said DNA vector.

The ratio of S.typhi cells to DNA vector in the immunogenic preparations according to the invention may be selected in conventional manner and may vary according to the S.typhi strain and particular type of DNA vector employed and mode of preparation of the preparations. However, the DNA vector is conveniently present in a proportion which provides, upon expression, 0.01 to 2.5μg of the LT-B toxin sub-unit of E.coli per $10^9$ cells of S.typhi.

In view of the generation of the immunogenic E.coli LT-B subunit by the DNA vector contained in the above-described preparations, the latter preparations may be employed in the treatment of E.coli diseases caused by the LT-toxin as opposed to the ST-toxin.

MG/TJM/8th May, 1984

The present invention also provides to above-described immunogenic preparations for use in the treatment or prophylaxis of typhoid and cholera or E.coli infections caused by the LT-toxin. The present invention further provides a method for the prophylaxis or treatment of typhoid and cholera or E.coli infections caused by the LT-toxin in a human or non-human animal subject which comprises administering to the said subject an effective dose of the above-described immunogenic preparation.

The live non-virulent S.typhi strain employed in the immunogenic preparations according to the present invention is conveniently a strain as described in UK Patent Specification 1291214.

The DNA vector that is used to transform the S.typhi strain advantageously contains a DNA nucleotide sequence coding for the LT-B toxin subunit of E.coli of human origin. This sequence is herein designated as $eltB_H$ while the corresponding sequence for material of porcine origin is designated $eltB_p$. The amino acid sequence analysis of $eltB_H$ has been described by Yamamoto et al, J. Bact., Oct. 1982, 506-509, which also refers to previous work involving the cloning of this gene.

The plasmid that is employed in accordance with the present invention also advantageously has a high copy number, e.g. between 15 and 50 and preferably about 30, and expresses substantial amounts of the LT-B toxin subunit, examples of such plasmids being described below.

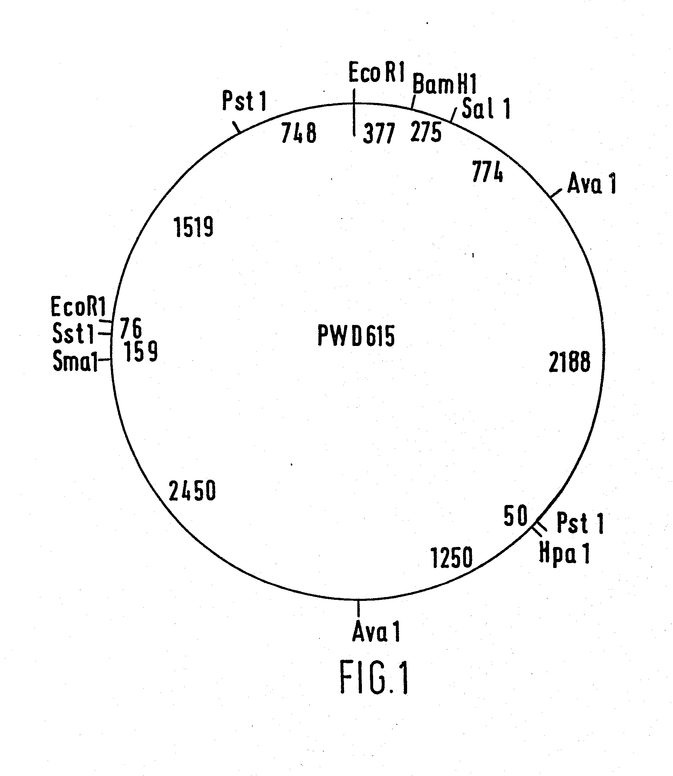

A particularly preferred plasmid containing the $eltB_H$ DNA sequence is that which is herein designated pWD615 and characterised and prepared as herein described. A restriction enzyme map of pWD615 is shown in Figure 1.

The plasmid pWD615 may be prepared from a plasmid designated herein as pWD600 by deletion of an XbaI site from the latter plasmid. pWD600 contains the cistrons eltA and eltB which code respectively for the A subunit and the B subunit of the E.coli LT toxin. Synthesis of the undesired LT-A subunit was therefore prohibited by introducing a frameshift mutation into eltA of pWD600 which did not prevent the transcription of mRNA encoding LT-B. A frameshift mutation was introduced into eltA at the unique XbaI site, located at amino acid codons 10 and 11. Deletion of the XbaI site from pWD600 entails loss of coding for LT-A subunit.

MG/TJM/8th May, 1984

The plasmid pWD600 contains an elt$_H$ DNA fragment derived from a human isolate of enterotoxigenic E.coli H74-114. Plasmid DNA was isolated from H74-114 in conventional manner and the plasmid was then cleaved with Pst I. The cleaved plasmid was then admixed with the plasmid pBR322 previously cleaved also with Pst I to obtain the recombinant plasmid pWD600. A method of preparation of pWD600 and pWD615 is described below by way of example.

The plasmids containing the eltB described above may be used to transform the S.typhi strain to provide bivalent immunogenic preparations in accordance with the invention. This transformation may be effected in conventional manner, e.g. as described by Lederberg & Cohen, J. Bact., 119, 1072-1074.

The present invention further provides vaccine formulations comprising an immunogenic preparation according to the invention together with at least one vaccine adjuvant or carrier.

The immunogenic preparations in accordance with the invention may be formulated as vaccine formulations in conventional manner, the particular procedure employed depending on the form of the immunogenic preparation that is employed. Thus, for example, for the formulation of a vaccine containing a live attenuated strain of S.typhi the immunogenic preparation is advantageously lyophilised. The lyophilised preparation may be presented for example in a capsule or tablet, e.g. gelatin capsules or capsules provided with an enteric coating comprising for example Eudragate"S", Eudragate"L", cellulose acetate, cellulose phthalate or hydroxypropylmethyl cellulose phthalate. Alternatively, the lyophilised material may be reconstituted prior to administration, e.g. as a suspension. Reconstitution is advantageously effected in a buffer of suitable pH to ensure the viability of the organisms, e.g. a buffer medium comprising disodium phosphate, monosodium phosphate and sucrose. In order to protect the live bacteria in the vaccine from gastric acidity, a sodium bicarbonate preparation is advantageously administered before each administration of the vaccine.

The rate of administration of the vaccine formulation will depend on various clinical factors, including the size and weight of the patient, the type of vaccine formulation employed etc. However, for a live avirulent S.typhi strain a dosage

MG/TJM/8th May, 1984

X234

0127153

regime comprising the administration of 3 dosage units of vaccine formulation each dosage unit containing at least $10^9$ e.g. $10^9$-$10^{10}$ S.typhi organisms, and a DNA vector expressing 0.01 to 25µg of the LT-B toxin sub-unit of E.coli, preferably every second day, is generally convenient.

The following Examples illustrate the present invention.

## Example 1

### Preparation of Plasmids and Transformation of S.Typhi

Bacterial strains and plasmids. The human isolate of enterotoxigenic E.coli H74-114 was employed (Rappaport R.S. 1978, Proceedings of the 13th Joint Conference of the U.S. Japan Cooperatie Medical Science Program Symposium on Cholera. Publ. No. (NIH) 78-1590. Department of Health, Education and Welfare, Washington D.C.). The K-12 strain, MM294 (ATCC. 33625 (endo $I^-$, $B_1^-$, $r_k^-$, $m_k^+$)), was used in all recombinant DNA experiments.

Salmonella typhi strain Ty21a which is commercially available under the trade mark VIVOTIF was obtained from R. Germanier of the Swiss Serum and Vaccine Institute, Berne, Switzerland; this strain may be obtained by the procedures described in UK Patent Specification 1291214.

Isolation of plasmids. Plasmid DNA from H74-114 was isolated as described by Portnoy et al for 100 ml cultures. (Infect. Immun. 31:775-782). Further purification was obtainned by caesium chloride centrifugation (final density 1.55 g/ml) in the presence of 400 µg/ml ethidium bromide for at least 3 hr at 70,000 rpm and $20^o$C using a Beckman VTi70 rotor. Plasmid DNA from MM294 transformants was isolated using a Tritonn X-100 cleared lysis method coupled with the caesium chloride (CsCl)/ethidium bromide (EtBr) centrifugation procedure already described. Plasmid bands were removed from gradients by puncturing the side of the centrifuge tube with a 16 gauge needle and syringe. EtBr was removed by four extractions with an equal volume of water-CsCl saturated isopropanol. CsCl was removed and the plasmid DNA concentrated by ethanol precipitation.

Preparation of PWD600. Approximately 14 µg of plasmid DNA from H74-114, isolated as described above, was cut with PstI and fractionated using a Bethesda Research Laboratories preparative electrophoresis system. Fractions with $elt_H$

DNA sequences (LT gene isolated from E.coli infections for humans) were identified by hybridization to a radiolabeled elt$_p$ DNA fragment (LT gene isolated from E.coli infections for piglets (Mosely S.L. et al, J.Infect. Dis. 145:863-869 (1982)) using a minifold filtrationn manifold from Schleicher and Schuell. To denature the DNA, 20 µl of each 450 µl fraction was mixed with 180 µl 0.5M NaOH. After incubating for 5 min. at room temperature, the solutions were transferred to nitrocellulose filter paper (NC) using the manifold. The NC was next rinsed with 1M Tris pH7 1.5M NaCl. The NC was then treated in the usual fashion for Southern hybridization. The five hybridization positive fractions were pooled and concentrated by ethanol precipitation. One fifth of the sample was added to 1 µg of PstI cleaved pBR322 and T4 ligase was added. The final volume of the reaction mixture was 30 µl. Plasmid DNA was introduced into MM294 as described by Lederberg and Cohen ibid. A recombinant plasmid, pWD600, derived from pBR322 and containing a DNA fragment with elt$_H$ was isolated and identified both by hybridisation and immunological detection of LT.

Preparation of pWD615. pWD600 was cleaved with XbaI which leaves four bases unpaired at both 5' ends of the duplex plasmid. Addition of Klenow fragment from DNA polymerase I catalyzed the addition of four bases at each end of the DNA. The net result was the addition of four extra nucleotide base pairs to the original plasmid and loss of the XbaI site. The ends of the DNA fragment were joined by T4 DNA ligase. A plasmid was identified which had lost the XbaI site (it was resistant to XbaI cleavage), did not confer upon the host bacteria the LT$^+$ phenotype (extracts were negative in a tissue culture cell assay that detects LT), but which did confer the LT-B$^+$ phenotype (by immunological assay). This plasmid was designated pWD615.

Transformation of Ty21a. An overnight culture of Ty21a was used to inoculate 50 ml of L-broth (1/100 dilution). Cells were grown to an optical density of 600 nm = 0.7. The culture was then harvested in a refrigerated centrifuge (4$^o$C, 3,000 rpm for 5 minutes in an ss34 rotor using a Sorval RC5-B centrifuge). Cells were gently resuspended in 50 ml of cooled 0.1M MgCl$_2$ and harvested again. Cells were gently resuspended in 50 ml 0.1M CaCl$_2$ and kept on ice for 20 minutes. Cells were harvested and gently resuspended in 2.5 ml 0.1M CaCl$_2$. For transformations, 0.2 ml samples of commpetent cells were transformed by 0.01 ml pWD615 (10 µg) in TE buffer (20 mM Tris pH8, 0.5 mM EDTA). pWD615

MG/TJM/8th May, 1984

was added to competent cells and left on ice for 30 minutes. The mixture was next put at 42$^{0}$C for 2 minutes and diluted 20-fold with prewarmed L-broth. After 90 minutes incubation period at 37$^{0}$C, the cells were concentrated to 1.0 ml by centrifugation and plated onto L-agar plates with 10 µg/ml tetracycline to test for tetracycline resistance.

The colonies which were resistant to tetracycline were the Ty21a colonies containing the pWD615 plasmid. These colonies were found to produce LT-B, as assayed by the G$_{MI}$-ELISA method described by Svennerholm and Holmgren, (1978, Curr. Microbiol. $\underline{1}$:19-23). A ganglioside mixture from P-L Biochemicals was used in place of purified G$_{MI}$ and the detecting reagents were a peroxidase (Zymed Laboratories) and 4-aminoantipyrine (Sigma).

## Example 2

### Vaccine Formulations

Vaccines for use in immunisation may be prepared by conventional techniques with the following constituents in which the immunogenic preparation, in accordance with the invention, contains at least 10$^{9}$ S.typhi cells and about 1µg of LT-B toxin sub-unit of E.coli.

a)    (i)     Gelatin Capsules

Each capsule (200 mg)
Immunogenic preparation

| | |
|---|---|
| Sucrose | 12-60 mg |
| Casein hydrolysate | 1.4-7 mg |
| Lactose | 100-180 mg |
| Magnesium stearate | 3.6-4.4 mg |
| Ascorbic acid | 1-5 mg |

(ii)     Sodium Bicarbonate Capsules

These should be taken 0.5 to 10 minutes before taking the capsules in (i). Each capsule (408 mg)

MG/TJM/8th May, 1984

| Sodium bicarbonate | 400 mg |
| Magnesium stearate | 8 mg |

b) **Enteric-Coated Capsules**

Capsules as prepared in a) (i) above but coated with an enteric coating of Eudragate "L", dissolving at a pH greater than 5.5 and applied using suitable apparatus. The capsules described in a) (ii) need not be taken with the capsules described in this example.

c) **Vaccine for Injection**

Each 1 ml of vaccine contains:-

| Immunogenic preparation | |
| Phenol | $<5.0$ mg |
| Sodium borate | $<10.98$ mg |
| Succinic acid | $<3.39$ mg |
| Sodium chloride | $<7.0$ mg |

d) **Freeze-Dried Capsules**

Each vial (3 ml) contains:-

| Immunogenic preparation (lyophilised) | |
| Sucrose | 12-60 mg |

The contents may be reconstituted with a suitable buffer e.g. saline solution.

**Example 3**

Table 1 illustrates the amount of LT-B toxin sub-unit produced when S.typhi strain Ty21a is transformed with pWD192 (a plasmid containing the $eltB_H$ cistron).

MG/TJM/8th May, 1984

X234
0127153

LT-B was determined in a $G_{M1}$-ganglioside-ELISA . (Enzyme-Linked Immunosorbent Assay). The control was performed by growing the untransformed Ty21a in parallel. The cells were disrupted with glass beads in a Sorval Omni and the cytoplasmic fraction was separated from cell wall and glass beads by centrifuging at 27,000 g and using the supernatant. LT-B associated with cell wall was not determined.

### Table 1

| Colony-Forming Units/ml | LT-B in Culture Supernatant µg/400ml | LT-B in Total µg | Cytoplasm µg/mg protein | Total LT-B in a 400ml Culture µg |
|---|---|---|---|---|
| $2 \times 10^8$ | 7.2 | 1.45 | 0.13 | 8.65 |
| $1.65 \times 10^9$ | 18.4 | 2.3 | 0.12 | 20.7 |
| $3.3 \times 10^9$ | 40 | 3.7 | 0.17 | 43.7 |
| $1.3 \times 10^8$ | 100 | 3.3 | 0.23 | 103.2 |

MG/TJM/8th May, 1984

X254CC

0127153

## Claims

1) Immunogenic preparations comprising a live, non-virulent strain of Salmonella typhi capable of providing protection against typhoid upon oral administration, together with a DNA vector which is capable of transforming said strain and which contains a sequence of bases which substantially encode the LT-B toxin sub-unit of E.coli.

2) Immunogenic preparations as claimed in claim 1 wherein the said strain of Salmonella typhi is Ty21a.

3) Immunogenic preparations as claimed in claim 1 or claim 2 wherein the DNA vector contains a sequence of bases which substantially encode the LT-B toxin sub-unit of E.coli of human origin.

4) Immunogenic preparations as claimed in claim 1 or claim 2 wherein the said DNA vector is the plasmid pWD615.

5) A method of preparing immunogenic preparations as claimed in any of claims 1 to 4 which comprises transforming the said strain of Salmonella typhi by the said DNA vector.

6) Vaccine formulations comprising an immunogenic preparation as claimed in any of claims 1 to 4 together with at least one vaccine adjuvant or carrier.

7) Vaccine formulations as claimed in claim 6 including a buffer.

8) Vaccine formulations as claimed in claim 6 or claim 7 in a lyophilised form.

9) Vaccine formulations as claimed in any of claims 6 to 8 in the form of dosage units.

10) Vaccine formulations as claimed in claim 9 wherein each dosage unit contains $10^9$ to $10^{10}$ Salmonella typhi organisms.

11) Immunogenic preparations as claimed in any of claims 1 to 4 for the treatment or prophylaxis of typhoid, cholera and E.coli diseases caused by the LT-toxin.

MG/TJM/8th May, 1984

FIG.1